# EUROPEAN PATENT SPECIFICATION

(11) **EP 4 093 478 B1**
(45) Date of publication and mention of the grant of the patent: **09.12.2026**
(21) Application number: 20707931.0
(22) Date of filing: 23.01.2020
(51) Int. Cl.: A61M 25/10

(54) **APPARATUS INCLUDING ENDOVASCULAR DEVICE CONNECTED TO A POWERED SYRINGE ASSEMBLY**
VORRICHTUNG MIT EINER AN EINE ANGETRIEBENE SPRITZENANORDNUNG ANGESCHLOSSENEN ENDOVASKULÄREN VORRICHTUNG
APPAREIL COMPRENANT UN DISPOSITIF ENDOVASCULAIRE RELIÉ À UN ENSEMBLE SERINGUE MOTORISÉ

(43) Date of publication of application: 30.11.2022
(73) Proprietor: C. R. Bard, Inc., Franklin Lakes, NJ 07417 (US)
(72) Inventor: TESSMER, Alexander, Phoenix, AZ 85018 (US)
(74) Representative: Hoffmann Eitle
(86) International application number: PCT/US2020/014778
(87) International publication number: WO 2021/150227

(56) References cited:
- EP-A1- 0 515 332
- EP-A1- 1 199 083
- US-A- 5 284 480
- US-A1- 2009 312 740
- US-A1- 2018 339 121

## Description

### TECHNICAL FIELD

This document relates generally to an apparatus, incorporating an endovascular device connected to a powered syringe assembly, that may be useful for percutaneous transluminal angioplasty, percutaneous transluminal valvuloplasty and other endovascular procedures, where rapid and precise balloon inflation and deflation or rapid and precise injection is desired.

### BACKGROUND

Percutaneous transluminal angioplasty or PTA refers to a minimally invasive medical procedure adapted to open blocked arteries or veins in order to allow improved blood flow to the muscles of the heart. Percutaneous transluminal valvuloplasty or PTV refers to a minimally invasive medical procedure adapted to repair a heart valve that has a narrowed or restricted opening.

PTA and PTV rely upon the use of a catheter incorporating a dilatation balloon. Currently, physicians use a syringe or an inflation device, that is pushed or twisted by hand to inflate the dilatation balloons of PTA and PTV catheters. Significantly, the physician must exert a tremendous amount of force with his or her hands to inflate the balloon quickly when using a syringe or inflation device. The force necessary also makes it very hard to control the inflation process with any precision.

Generally, physicians performing these procedures would like the ability to inflate and deflate the dilatation balloon on the catheter quickly and in a controlled manner. This is particularly of interest during PTV procedures since the inflated balloon obstructs blood flow through the ventricle. For example, during an aortic valvuloplasty procedure the inflated balloon obstructs the flow of blood discharged through the left ventricle until the balloon is deflated enough for the physician to pull the PTV dilatation catheter back into the aorta.

This document discloses a new and improved apparatus that incorporates an endovascular device connected to a configured-to-be-powered syringe assembly that allows for controlled and rapid retraction or withdrawal of the plunger from the syringe body of the powered syringe assembly. This is a substantial benefit when performing PTA and PTV medical procedures as it provides the physician with user friendly and precise control of the balloon inflation and deflation process. Such an apparatus is also useful for other procedures involving, for example, (a) the injection of contrast agents and/or saline mixtures through an introducer sheath or catheter for visualization during fluoroscopy, (b) drug or therapeutic agent delivery via a porous balloon or catheter, (c) aspiration of thrombus (blood clot) through a sheath or catheter, and (d) delivery of radioactive beads, drug coated beads, embolic beads, embolic particles or glue through a catheter. In general, the new and improved apparatus is useful for injection of any desired material inside of the body and aspiration of any desired material from the body including, particularly, the vasculature of the body.
EP 1 199 083 A1 discloses an apparatus comprising, in a single, transportable body provided with a handle, a support for a motor-driven impelling syringe unit connected to a pressure tube, forming a single-use, replaceable element, driven by means of a motor and helical-screw unit the motor of which is operated by means of a control board which receives information relating to the force of the piston of the motor-driven syringe, its positioning, and also acoustic (or phonetic) instructions from a microphone, and electronic instructions from a touch screen which can display the parameters indicative of the progress of the operation for the correction a lesion of an organic duct (artery, vein, bile duct, urological duct, etc.) as well as receiving instructions from the operator.
US 5 284 480 A discloses an inflation device for manual inflation of a balloon of a balloon catheter.
US 2018/339121 A1 discloses a cuff pressure adjusting device.

### SUMMARY

In accordance with the present invention, the purposes and benefits set forth herein, a new and improved apparatus according to claim 1 is provided.

Preferred embodiments are set forth in the dependent claims.

Associated surgical methods are also described herein to aid understanding the invention. These methods do not form part of the claimed invention. That apparatus is particularly suited for application in PTA and PTV medical procedures as well as other medical procedures, such as previously listed, where controlled, rapid retraction and withdrawal of the plunger in a syringe body is desired.

According to the present invention, an apparatus comprises
an endovascular device; and
a configured-to-be-powered syringe assembly connected to the endovascular device,
wherein the syringe assembly comprises a reusable drive unit including a drive motor and a housing, wherein the housing is a handheld housing which is ergonomically adapted to be held in the hand of a physician; and
a disposable syringe unit, wherein the housing includes a releasable lid and an internal compartment for receiving and holding the disposable syringe unit and the drive motor.

The apparatus comprises an endovascular device and a syringe assembly connected to that endovascular device. That syringe assembly is configured for powered operation.

The endovascular device refers to a device at least partially inserted inside the blood vessel of a patient to perform a medical procedure. For purposes of this document the terminology "endovascular device" includes a catheter, an angiographic catheter, a diagnostic catheter, a guiding catheter, a microcatheter, a specialty catheter, a sheath, an angioplasty balloon, a drug coated balloon, a specialty balloon, an embolectomy balloon, a valvuloplasty balloon, a balloon expandable stent, a self-expanding stent, a balloon expandable covered stent, a self-expanding covered stent, a drug delivery device, an embolization device, an atherectomy device, a CTO crossing device, a thrombectomy device, a contrast management device, a contrast injection line, a thrombus aspiration device, a dialysis catheter, a central line catheter, a peripheral IV catheter, a PICC line and an endoscope.

The configured-to-be-powered syringe assembly includes a reusable drive unit and a disposable syringe unit. More particularly, the reusable drive unit includes a housing and a drive motor having a pinion. The disposable syringe unit includes a syringe body, a plunger received in the syringe body, a spindle nut carried on the syringe body and a threaded spindle carried on the plunger wherein the disposable syringe unit is held in the housing and the pinion meshes with and drives the spindle nut in a first direction to retract the plunger into the syringe body and in a second direction to withdraw the plunger from the syringe body.

In one or more of the many possible embodiments of the apparatus, the spindle nut includes an integral gear that meshes with the pinion.

In one or more of the many possible embodiments of the apparatus, the disposable syringe unit further includes a tube extending from the syringe body to a first connector and the endovascular device includes a second connector at the distal end whereby the first connector may be connected to establish a fluid pathway between the syringe body and the balloon. The connection between the endovascular device and the syringe assembly may be by a standard luer lock connection.

In one or more of the many possible embodiments of the apparatus, the housing is a handheld housing: that is, a housing ergonomically adapted to be held in the hand of a physician. In one or more of the many possible embodiments of the apparatus, the housing includes at least one actuator for the drive motor. In one or more of the many possible embodiments of the apparatus, the at least one actuator includes a first push button to retract the plunger from the syringe body and a second push button to withdraw the plunger from the syringe body. In one or more of the many possible embodiments of the apparatus, the housing includes a speed switch for manually adjusting an operating speed of the drive motor.

In one or more of the many possible embodiments of the apparatus, the housing includes a digital display for displaying operating information respecting the apparatus. That digital display may display a balloon inflation pressure or other data of interest to the physician such as the volume of fluid forced from or drawn into the syringe body by displacement of the plunger.

In one or more of the many possible embodiments of the apparatus, the apparatus includes a controller configured to control operation of the drive motor and, as a consequence, the retraction and withdrawal of the plunger with respect to the syringe body. The controller may also be configured to control operation of the digital display.

In one or more of the many possible embodiments of the apparatus, the drive motor is an electric drive motor. In such embodiments, the apparatus may further include a battery carried on the housing and adapted to power the drive motor. In one or more of the many possible embodiments of the apparatus, the drive motor is a pneumatic motor. In such embodiments, the pneumatic motor may be connected to an air source remote from the housing or handle.

In one or more of the many possible embodiments of the apparatus, the housing includes a releasable lid and an internal compartment for receiving and holding the disposable syringe unit and the drive motor and pinion. In one or more of the many possible embodiments of the apparatus, the housing may include a seat for holding the syringe body in position within the housing while the plunger is displaced.

In accordance with an additional embodiment, an apparatus comprises: (a) a catheter having a proximal end and a distal end, (b) a balloon carried on the catheter adjacent the distal end and (c) a syringe assembly configured for powered operation. That configured-to- be-powered syringe assembly may include a reusable drive unit and a disposable syringe unit. The reusable drive unit may include a housing and a drive motor for driving a pinion. The drive motor and pinion are held in the housing. The disposable syringe unit may include a syringe body, a plunger received in the syringe body, a spindle nut carried on the syringe body and a threaded spindle carried on the plunger.

The disposable syringe unit may be held in the housing with the drive motor.
The pinion meshes with and drives the spindle nut (a) in a first direction to retract the plunger into the syringe body and inflate the balloon and (b) in a second direction to withdraw the plunger from the syringe body and deflate the balloon.

The syringe assembly may also include a controller, that may be carried on the housing, configured to control operation of the reusable drive unit and, as a consequence thereof, the inflation and deflation of the balloon.

In accordance with a non-claimed example, a new and improved method of treating a patient is provided. That method comprises the steps of: (a) positioning a balloon carried on a catheter at a target site in a vasculature of the patient, (b) selectively inflating the balloon with a configured-to-be-powered syringe assembly including a disposable syringe unit and a reusable drive unit adapted for displacing a plunger of the disposable syringe unit; (c) remediating an occlusion of the vasculature of the patient and (d) selectively deflating the balloon with the powered syringe assembly. For purposes of this document, "vasculature" refers to the blood vessels, including the coronary blood vessels, and the heart.

The method may further include controlling, by controller, the configured-to- be-powered syringe assembly and thereby the inflating and deflating of the balloon. The method may also include the step of removing the catheter from the vasculature of the patient.

With regard to the remediating step, that step may include opening a blood vessel including, but not necessarily limited to a coronary artery, a brain artery and a pulmonary artery, and/or opening a narrowed heart valve of the patient. Still further, the method may include the step of controlling, by controller, operation of the syringe assembly and thereby the inflating and deflating of the balloon.

In other examples, a method of treating a patient includes the steps of: (a) positioning an endovascular device in a vasculature of the patient and (b) selectively delivering a syringe injectable material into the vasculature with a configured-to-be-powered syringe assembly including a disposable syringe unit and a reusable drive unit adapted for displacing a plunger of the disposable syringe unit.

That method may further include the step of selecting said syringe injectable material from a group of materials consisting of a contrast agent, a saline solution, a therapeutic agent, radioactive beads, embolic beads, embolic particles, glue and combinations thereof.

In yet another possible example, a method of treating a patient includes steps of:
(a) positioning an endovascular device in a vasculature of the patient and (b) selectively aspirating a material from the patient with a configured-to-be-powered syringe assembly connected to the endovasculature device wherein the powered syringe assembly includes a disposable syringe unit and a reusable drive unit adapted for displacing a plunger of the disposable syringe unit.

In the following description, there are shown and described several preferred embodiments of the apparatus and examples of a method of treating a patient. The drawing figures and descriptions should be regarded as illustrative in nature rather than descriptive.

### BRIEF DESCRIPTION OF THE DRAWING FIGURES

The accompanying drawing figures incorporated herein and forming a part of the specification, illustrate several aspects of the catheter system and together with the description serve to explain certain principles thereof.
Figure 1 is a perspective view of one possible embodiment of the apparatus including a catheter, a balloon mounted on the catheter and a syringe assembly that is configured for powered operation.
Figure 2 is a detailed perspective view of the powered syringe assembly shown in Figure 1 with the releasable lid thereof opened, the disposable syringe unit exploded from the internal compartment of the housing and the reusable drive unit held in the internal compartment of the housing.
Figures 3A-3C are a series of detailed side elevational views of the disposable syringe unit illustrating the plunger thereof in respective fully retracted, partially retracted and fully withdrawn positions.
Figure 4 is a schematic illustration of the controller that controls the operation of the drive motor that drives the displacement of the plunger within the syringe body and as a consequence thereof, the inflation and deflation of the catheter mounted balloon illustrated in Figure 1.

Reference is now made in detail to the catheter system, example embodiments of which are illustrated in the accompanying drawing figures.

### DETAILED DESCRIPTION

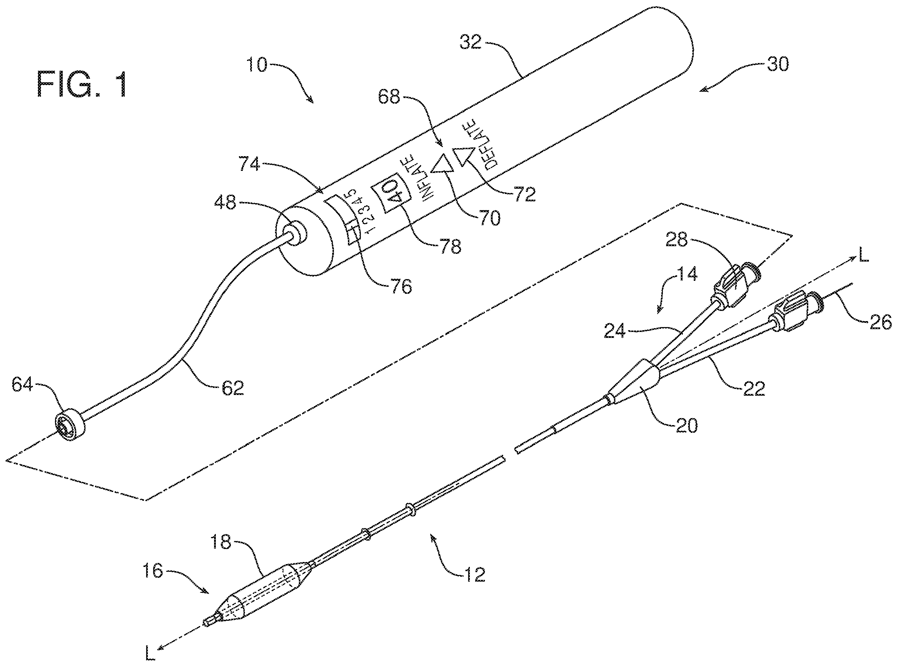

Reference is now made to Figures 1-4 illustrating the new and improved apparatus 10. That apparatus 10 illustrated in the drawing figures takes the form of a catheter system particularly adapted for application in various medical procedures including percutaneous transluminal angioplasty (PTA), percutaneous transluminal valvuloplasty (PTV) and other procedures, such as, but no limited to, peripheral and coronary angioplasty, where rapid and precise balloon inflation and deflation is desired.

As illustrated, the apparatus 10 includes an endovascular device in the form of a catheter 12 having an elongated body with (a) a longitudinal axis L, (b) a proximal end 14 and (c) a distal end 16. A balloon 18 is carried on the catheter 12 adjacent the distal end 16.

The catheter 12 may be made from an extruded polymer material (e.g.
PEBAX^{®} brand polyether block amide (PEBA), Nylon, polyethylene terephthalate (PET), etc.) which may or may not be braided or loaded with radioopaque materials. The catheter shaft may also be a stainless steel or nitinol hypotube with or without cuts to increase flexibility which may or may not be laminated with various types of plastic (PEBAX^{®} brand PEBA, Nylon, PET, etc.). The catheter 12 may be of considerable length (e.g. 40-300 centimeters, or otherwise suitable to allow the distal end 16 of the catheter to reach a treatment area of interest in the vasculature of a patient while the proximal end 14 of the catheter remains accessible to the surgeon external to the vasculature.

The balloon 18 may be compliant or noncompliant and made from any appropriate material known in the art to be suitable for the indicated purpose or medical procedure. The inflatable balloon 18 may or may not be braided, may or may not include multiple layers of material and may or may not include reinforcing fibers. The balloon 18 may be coated with a therapeutic agent, such as anti-stenosis or anti-proliferation drugs including sirolimus, paclitaxel, zotarolimus, everolimus, Biolimus A9 and the like. In one or more of the many possible embodiments, the balloon 18 may be a vibration balloon such as disclosed in U.S. Patent 10,245,051 issued to Bard Peripheral Vascular, Inc. of Tempe, Arizona.

As illustrated in Figure 1, the proximal end 14 of the catheter 12 may include a connector 20 having one or more ports. More particularly, in the illustrated embodiment, the connector 20 includes a first port 22 and a second port 24. The first port 22 may receive a guide wire 26 for use as an aid in positioning the catheter 12 and, more particularly the balloon 18 carried thereon at a desired target position within the aorta, heart or other anatomical structure of the patient in a manner known in the art. A luer lock 28 at the second port 24 may allow connection of the connector 20 to a syringe assembly, generally designated by reference numeral 30. That syringe assembly 30 is configured for powered operation.

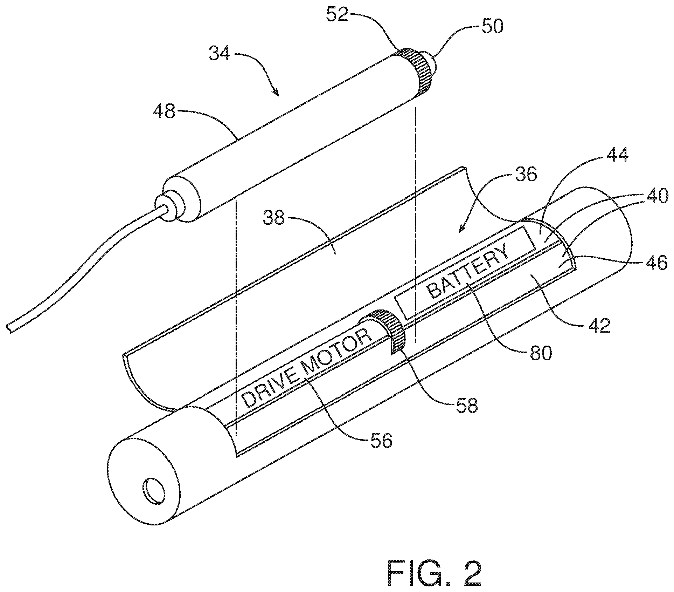

The illustrated configured-to-be-powered syringe assembly 30 includes a disposable syringe unit 34 and a reusable drive unit 36. More particularly, the reusable drive unit 36 includes a housing 32 having a releasable lid 38, that may be pivoted open as illustrated in Figure 2 in order to access an internal compartment 40 of the housing. As illustrated, that internal compartment 40 includes a partition 42 dividing the internal compartment into a first section 44 adapted to receive and hold the drive motor 56 of the reusable drive unit 36 and a second section 46 adapted to receive and hold the disposable syringe unit 34.

In the illustrated embodiment, the disposable syringe unit 34 includes a syringe body 48, a plunger 50 received for sliding movement within the syringe body 48, a spindle nut 52 carried on the syringe body and adapted to freely rotate with respect thereto and a threaded spindle 54 carried on the plunger 50.

In the illustrated embodiment, the reusable drive unit 36 includes the drive motor 56 having a pinion 58. When the disposable syringe unit 34 and the drive motor 56 are properly mounted and held in the housing 32, the pinion 58 meshes with and drives the spindle nut 52 in a first direction to retract the plunger 50 into the syringe body 48 and inflate the balloon 18 and in a second direction to withdraw the plunger from the syringe body and deflate the balloon. More specifically, the spindle nut 52 includes or comprises an integral gear that freely rotates with respect to the syringe body 48 and meshes with the pinion at a gap 60 formed in the partition 42.

The disposable syringe unit 34 further includes a tube 62 extending from an opening 63 in the end of the syringe body 48 to a connector 64. That first connector 64 is connected to the second connector 20 at the luer lock 28. This establishes a fluid pathway between the syringe body 48 and the balloon 18 through the second port 24 and a dedicated balloon communication conduit (not shown) in the catheter 12.

As should be further appreciated from viewing Figure 1, the housing 32 takes the form of a handheld housing or handle; that is, a housing ergonomically adapted to be comfortably held in the hand of a physician. As illustrated in Figure 1, that housing 32 includes at least one actuator 68 for the drive motor 56. More particularly, in the illustrated embodiment the actuator 68 includes a first push button 70 that is depressed in order to inflate the balloon 18 and a second push button 72 that is depressed in order to deflate the balloon.

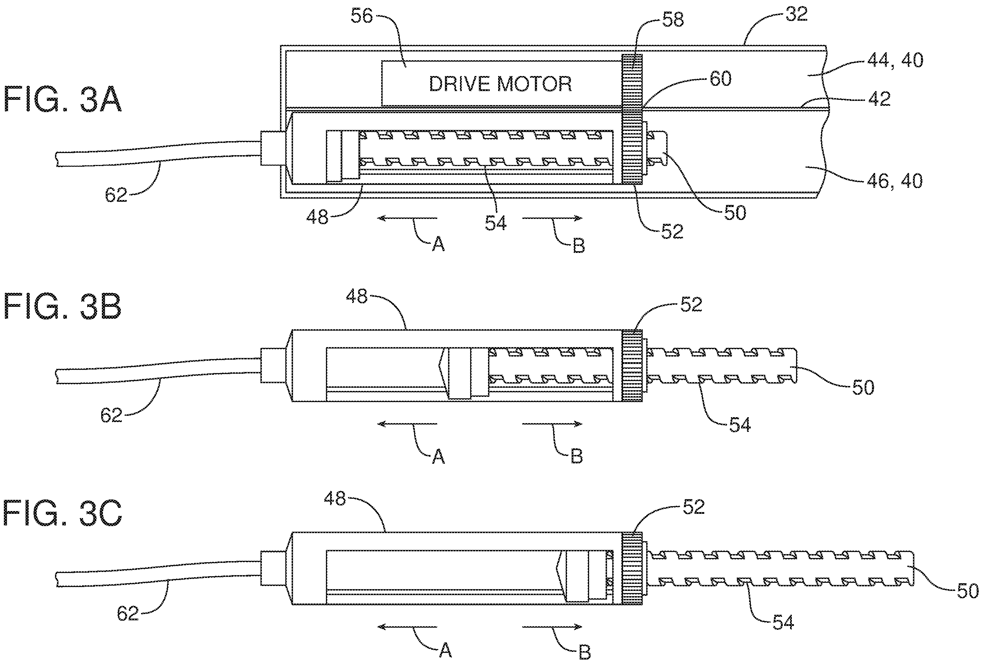

When the physician depresses the first push button 70, the drive motor 56 drives the pinion 58 that, in turn, drives the spindle nut 52 in a first direction. The spindle nut 52 meshes with the threaded spindle 54 of the plunger 50 causing the plunger 50 to be displaced in the direction of action arrows A from, for example, the fully withdrawn position illustrated in Figure 3C through the partially retracted position illustrated in Figure 3B to the fully retracted position illustrated in Figure 3A. As this is done, the plunger 50 forces the working fluid, of a type known in the art, in the syringe body from the syringe body 48 through the tube 62 and the dedicated balloon communication conduit in the catheter 12 into the balloon 18 so as to cause inflation of the balloon.

In contrast, when the physician depresses the second push button 72, the drive motor 56 drives the pinion 58 that, in turn, drives the spindle nut 52 in a second direction. The spindle nut 52 meshes with the threaded spindle 54 of the plunger 50 causing the plunger 50 to be displaced in the direction of action arrows B from, for example, the fully retracted position illustrated in Figure 3A through the partially retracted position illustrated in Figure 3B to the fully withdrawn position illustrated in Figure 3C. As this is done, the plunger 50 draws the working fluid in the balloon 18 through the dedicated balloon communication conduit in the catheter 12 and the tube 62 into the syringe body 48 so as to cause deflation of the balloon.

As should be appreciated, the second section 46 of the internal compartment
40 acts as a seat for holding the syringe body 48 in place within the housing 32 as the plunger 50 is displaced to inflate and deflate the balloon 18. Tabs, lugs, ribs or other internal structures of a type known in the art, but not illustrated, can be used for this purpose.

The illustrated embodiment of the catheter system 10 also includes an optional speed switch 74 for manually adjusting the operating speed of the drive motor 56 that allows a physician to adjust and control the speed of inflation and deflation of the balloon 18. In the illustrated embodiment, the speed switch 74 includes a control tab 76 that may be manipulated into any one of five predetermined speed positions. Other embodiments may include a different number of predetermined speed positions or none at all.

In the illustrated embodiment, the housing 32 also includes a digital display 78 adapted to display operating information respecting the apparatus 10. For example, the digital display 78 may display current balloon inflation pressure or other information of interest to the physician including, but not necessarily limited to the volume of fluid being expelled from the syringe body into the balloon or withdrawn from the balloon into the syringe body.

In one possible embodiment of the apparatus 10, the drive motor 56 is an electric drive motor. In such an embodiment, the drive motor 56 may be powered by a suitable battery 80 carried in the first section 44 of the housing 32. Alternatively or in addition, the drive motor 56 may be connected to a remote source of electrical power by means of a cord (not shown) extending from the housing 32.

In another possible embodiment of the apparatus 10, the drive motor 56 is a pneumatic motor that may be connected to a remote source of pneumatic power, such as an air source (not shown) by means of a hose connection (not shown).

As illustrated in Figure 4, the apparatus 10 may also include a controller 82 configured or adapted to automatically control operation of the drive motor 56 and as a consequence, provide precisely controlled inflation and deflation of the balloon 18. As illustrated in Figure 4, the controller 82 may be connected to the battery 80, the actuator 68, the drive motor 56, the digital display 78 (if present) and the speed switch 74 (if present).

Such a controller 82 may comprise a computing device in the form of a dedicated microprocessor or an electronic control unit (ECU) operating in accordance with instructions from appropriate control software. The controller 82 may comprise one or more processors, one or more memories and one or more network interfaces in communication with each other over one or more communication buses. The controller 82 may be carried in the internal compartment 40 of the housing 32 or may be remote from the housing 32 and be in wireless or hardwired communication with the drive motor 56, the actuator 68, the digital display 78 (if present) and the speed switch 74 (if present), within the housing.

Such a "smart" apparatus 10 of the type described is particularly advantageous in PTA procedures where the effectiveness of a drug-eluting balloon 18 often depends on the pressure with which the balloon is inflated and/or the length of time the inflated balloon contacts the vessel wall. The controller 82 optimizes these parameters for best effectiveness. Similarly, in PTV procedures the effectiveness of the balloon 18 also often depends upon the pressure with which the balloon is inflated and/or the length of time the inflated balloon contacts the valve opening. Again, the controller 82 optimizes these parameters for more effective treatment.

As should be appreciated, the apparatus 10 may be used in a method of treating a patient (the method not claimed). That method includes the steps of: (a) positioning a balloon 18 carried on a catheter 12 at a target site in a vasculature of the patient, (b) selectively inflating the balloon with a configured-to-be-powered syringe assembly 30 including a disposable syringe unit 34 and a reusable drive unit 36 adapted for displacing a plunger 50 of the disposable syringe unit; (c) remediating an occlusion of the vasculature of the patient and (d) selectively deflating the balloon with the syringe assembly.

Further, the method may include the step of controlling, by controller 82, the operation of the syringe assembly 30 and, thereby, the inflating and deflating of the balloon 18 in a precise manner engineered to optimize the effectiveness of the patient treatment. In addition, the rediating step may include opening (a) a blood vessel or (b) a narrowed heart valve of the patient.

Still further, the method of treating a patient may include the steps of: (a) positioning an endovascular device in a vasculature of a patient and (b) selectively delivering a syringe injectable material into the vasculature with a configured-to-be-powered syringe assembly 30 including a disposable syringe unit 34 and a reusable drive unit 36 adapted for displacing a plunger 50 of the disposable drive unit. The syringe injectable material may comprise a wide range of materials including, but not necessarily limited to: (a) contrast agents and contrast and saline mixtures through an introducer sheath or a catheter for visualization during fluoroscopy, (b) therapeutic agent or drug delivery via a porous balloon or catheter, (c) delivery of radioactive beads, drug coated beads, embolic beads, embolic particles or glue through a catheter and (d) aspiration of thrombus (blood clot) through a sheath or catheter. More generally, the method is useful for injecting medical procedure materials or agents into the vasculature or aspirating materials from the body. This also includes injection or aspiration through an endoscope during a medical procedure by connection of the endoscope to the powered syringe assembly as previously described.

Each of the following terms written in singular grammatical form: "a", "an", and the", as used herein, means "at least one", or "one or more". Use of the phrase One or more" herein does not alter this intended meaning of "a", "an", or "the". Accordingly, the terms "a", "an", and "the", as used herein, may also refer to, and encompass, a plurality of the stated entity or object, unless otherwise specifically defined or stated herein, or, unless the context clearly dictates otherwise. For example, the phrases: "a unit", "a device", "an assembly", "a mechanism", "a component, "an element", and "a step or procedure", as used herein, may also refer to, and encompass, a plurality of units, a plurality of devices, a plurality of assemblies, a plurality of mechanisms, a plurality of components, a plurality of elements, and, a plurality of steps or procedures, respectively.

Each of the following terms: "includes", "including", "has", "having", "comprises", and "comprising", and, their linguistic / grammatical variants, derivatives, or/and conjugates, as used herein, means "including, but not limited to", and is to be taken as specifying the stated component(s), feature(s), characteristic(s), parameter(s), integer(s), or step(s), and does not preclude addition of one or more additional component(s), feature(s), characteristic(s), parameter(s), integer(s), step(s), or groups thereof. Each of these terms is considered equivalent in meaning to the phrase "consisting essentially of." Each of the phrases "consisting of and "consists of, as used herein, means "including and limited to". The phrase "consisting essentially of' means that the stated entity or item (system, system unit, system sub-unit device, assembly, sub-assembly, mechanism, structure, component element or, peripheral equipment utility, accessory, or material, method or process, step or procedure, sub-step or sub-procedure), which is an entirety or part of an exemplary embodiment of the disclosed invention, or/and which is used for implementing an exemplary embodiment of the disclosed invention, may include at least one additional feature or characteristic" being a system unit system sub-unit device, assembly, sub-assembly, mechanism, structure, component or element or, peripheral equipment utility, accessory, or material, step or procedure, sub-step or sub-procedure), but only if each such additional feature or characteristic" does not materially alter the basic novel and inventive characteristics or special technical features, of the claimed item.

The term "method", as used herein, refers to steps, procedures, manners, means, or/and techniques, for accomplishing a given task including, but not limited to, those steps, procedures, manners, means, or/and techniques, either known to, or readily developed from known steps, procedures, manners, means, or/and techniques, by practitioners in the relevant field(s) of the disclosed invention.

Terms of approximation, such as the terms about, substantially, approximately, etc., as used herein, refers to ± 10 % of the stated numerical value. Use of the terms parallel or perpendicular is meant to mean approximately meeting this condition, unless otherwise specified.

It is to be fully understood that certain aspects, characteristics, and features of the catheter system and method, which are, for clarity, illustratively described and presented in the context or format of a plurality of separate embodiments, may also be illustratively described and presented in any suitable combination or sub-combination in the context or format of a single embodiment. Conversely, various aspects, characteristics, and features, of the invention which are illustratively described and presented in combination or sub combination in the context or format of a single embodiment may also be illustratively described and presented in the context or format of a plurality of separate embodiments.

The foregoing has been presented for purposes of illustration and description. It is not intended to be exhaustive or to limit the embodiments to the precise form disclosed. Obvious modifications and variations are possible in light of the above teachings. For example, the catheter may include a handle at the proximal end. A strain relief of a type known in the art may be provided between the catheter 12 and such a handle. The strain relief may be an extrusion of various types of plastic such as PEBAX^{®} brand PEBA, Nylon, PET, etc. The apparatus 10 may include more ports than the two ports 22, 24 illustrated. As another example, the shaft of the catheter 12 may include Geo Align markings. As still another example, the apparatus 10 may include both the manual speed control 74 and the controller 82, the manual speed control without the controller or the controller without the manual speed control.

As a further example, the endovascular device is not limited to the illustrated balloon catheter but may comprise any one of the following: a catheter, an angiographic catheter, a diagnostic catheter, a guiding catheter, a microcatheter, a specialty catheter, a sheath, an angioplasty balloon, a drug coated balloon, a specialty balloon, an embolectomy balloon, a valvuloplasty balloon, a balloon expandable stent, a self-expanding stent, a balloon expandable covered stent, a self-expanding covered stent, a drug delivery device, an embolization device, an atherectomy device, a CTO crossing device, a thrombectomy device, a contrast management device, a contrast injection line, a thrombus aspiration device, a dialysis catheter, a central line catheter, a peripheral IV catheter, a PICC line and an endoscope. Further, the powered syringe assembly connected to the endovascular device may be used for a wide range of applications other than inflating and deflating a dilatation balloon including, but not necessarily limited to injecting contrasting agents, saline, therapeutic agents and various treatment beads and particulates as well as medical glue and selectively aspirating materials such as blood clots from the vasculature of the patient.

The scope of the present invention is defined by the appended claims.

## Claims

1. An apparatus (10), comprising:
an endovascular device; and
a configured-to-be-powered syringe assembly (30) connected to the endovascular device
**characterized in that**
the syringe assembly (30) comprises
a reusable drive unit (36) including a drive motor (56) and a housing (32), wherein the housing (32) is a handheld housing which is ergonomically adapted to be held in the hand of a physician; and
a disposable syringe unit (34), wherein the housing (32) includes a releasable lid (38) and an internal compartment (40) for receiving and holding the disposable syringe unit (34) and the drive motor (56).

2. The apparatus (10) of claim 1, **characterized in that** (a) the reusable drive unit (36) includes a drive motor (56) having a pinion (58), wherein the drive motor (56) and pinion (58) are held in the housing (32) and (b) the disposable syringe unit (34) includes a syringe body (48), a plunger (50) received in the syringe body (48), a spindle nut (52) carried on the syringe body (48) and a threaded spindle (54) carried on the plunger (50) wherein the disposable syringe unit (34) is held in the housing (32) and the pinion (58) meshes with and drives the spindle nut (52) in a first direction to retract the plunger (50) into the syringe body (48) and in a second direction to withdraw the plunger (50) from the syringe body (48).

3. The apparatus (10) of any of the preceding claims **characterized in that** said endovascular device is selected from a group of devices consisting of a catheter (12), an angiographic catheter, a diagnostic catheter, a guiding catheter, a microcatheter, a specialty catheter, a sheath, an angioplasty balloon, a drug coated balloon, a specialty balloon, an embolectomy balloon, a valvuloplasty balloon, a balloon expandable stent, a self-expanding stent, a balloon expandable covered stent, a self expanding covered stent, a drug delivery device, an embolization device, an atherectomy device, a CTO crossing device, a thrombectomy device, a contrast management device, a contrast injection line, a thrombus aspiration device, a dialysis catheter, a central line catheter, a peripheral IV catheter, a PICC line and an endoscope.

4. The apparatus (10) of claim 2, **characterized in that** the spindle nut (52) includes an integral gear that meshes with the pinion (58).

5. The apparatus (10) of any of the preceding claims, **characterized in that** the disposable syringe unit (34) further includes a tube (62) extending from the syringe body (48) to a first connector (64) and the endovascular device includes a second connector (20) at the distal end (16) thereof whereby the first connector (64) and the second connector (20) may be connected to establish a fluid pathway between the syringe body (48) and the endovascular device.

6. The apparatus (10) of any of the preceding claims, **characterized in that** the housing (32) includes at least one actuator (68) for the drive motor (56).

7. The apparatus (10) of any of the preceding claims, **characterized in that** the housing (32) includes a speed switch (74) for adjusting an operating speed of the drive motor (56).

8. The apparatus (10) of any of the preceding claims, **characterized in that** the housing (32) includes a digital display (78) for displaying operating information respecting the apparatus (10),
optionally, if dependent on claim 2, **characterized in that** the apparatus (10) further includes a controller (82) configured to control operation of the drive motor (56) and the digital display (78).

9. The apparatus (10) of claim 8, **characterized in that** the digital display (78) displays at least one of a balloon inflation pressure and a volume of liquid injected from the syringe body (48).

10. The apparatus (10) of claim 2, **characterized in that** said at least one actuator (68) includes a first push button (70) to retract the plunger (50) and a second push button (72) to withdraw the plunger (50).

11. The apparatus (10) of claim 2, **characterized in that** the drive motor (56) is an electric motor.

12. The apparatus (10) of claim 11, **characterized in that** the apparatus (10) further includes a battery (80) carried on the housing (32) and adapted for powering the electric motor.

13. The apparatus (10) of claim 2, **characterized in that** the drive motor (56) is a pneumatic motor.

14. The apparatus (10) of claim 2, **characterized in that** the housing (32) includes a seat for holding the syringe body (48) in place within the housing (32) while the plunger (50) is displaced in the syringe body (48).

15. The apparatus (10) of claim 2, **characterized in that** the apparatus (10) further includes a controller (82) configured to control operation of the drive motor (56) and as a consequence, retraction or withdrawal of the syringe with respect to the syringe body (48).

## Patentansprüche

1. Einrichtung (10), umfassend:
eine endovaskuläre Vorrichtung; und
eine für den angetriebenen Betrieb ausgebildete Spritzenanordnung (30), die an die endovaskuläre Vorrichtung angeschlossen ist
**dadurch gekennzeichnet, dass**
die Spritzenanordnung (30) Folgendes umfasst
eine wiederverwendbare Antriebseinheit (36), die einen Antriebsmotor (56) und ein Gehäuse (32) einschließt, wobei das Gehäuse (32) ein handgehaltenes Gehäuse ist, das ergonomisch angepasst ist, in der Hand eines Arztes gehalten zu werden; und
eine Einweg-Spritzeneinheit (34), wobei das Gehäuse (32) einen lösbaren Deckel (38) und ein inneres Fach (40) zum Aufnehmen und Halten der Einweg-Spritzeneinheit (34) und des Antriebsmotors (56) einschließt.

2. Einrichtung (10) nach Anspruch 1, **dadurch gekennzeichnet, dass** (a) die wiederverwendbare Antriebseinheit (36) einen Antriebsmotor (56) mit einem Ritzel (58) einschließt, wobei der Antriebsmotor (56) und das Ritzel (58) in dem Gehäuse (32) gehalten sind, und (b) die Einweg-Spritzeneinheit (34) einen Spritzenkörper (48), einen in dem Spritzenkörper (48) aufgenommenen Kolben (50), eine an dem Spritzenkörper (48) angeordnete Spindelmutter (52) und eine an dem Kolben (50) angeordnete Gewindespindel (54) einschließt, wobei die Einweg-Spritzeneinheit (34) in dem Gehäuse (32) gehalten ist und das Ritzel (58) mit der Spindelmutter (52) kämmt und diese in eine erste Richtung antreibt, um den Kolben (50) in den Spritzenkörper (48) zurückzuziehen, und in eine zweite Richtung, um den Kolben (50) aus dem Spritzenkörper (48) herauszuziehen.

3. Einrichtung (10) nach einem der vorstehenden Ansprüche, **dadurch gekennzeichnet, dass** die endovaskuläre Vorrichtung aus einer Gruppe von Vorrichtungen ausgewählt ist, bestehend aus einem Katheter (12), einem angiographischen Katheter, einem diagnostischen Katheter, einem Führungskatheter, einem Mikrokatheter, einem Spezialkatheter, einer Schleuse, einem Angioplastieballon, einem wirkstoffbeschichteten Ballon, einem Spezialballon, einem Embolektomieballon, einem Valvuloplastieballon, einem ballonexpandierbaren Stent, einem selbstexpandierenden Stent, einem ballonexpandierbaren Stentgraft, einem selbstexpandierenden Stentgraft, einer Wirkstoffabgabevorrichtung, einer Embolisationsvorrichtung, einer Atherektomievorrichtung, einer CTO-Durchquerungsvorrichtung, einer Thrombektomievorrichtung, einer Kontrastmittelmanagementvorrichtung, einer Kontrastmittelinjektionsleitung, einer Thrombusaspirationsvorrichtung, einem Dialysekatheter, einem Zentralvenenkatheter, einem peripheren intravenösen Katheter, einem PICC-Katheter und einem Endoskop.

4. Einrichtung (10) nach Anspruch 2, **dadurch gekennzeichnet, dass** die Spindelmutter (52) ein integrales Zahnrad einschließt, das mit dem Ritzel (58) kämmt.

5. Einrichtung (10) nach einem der vorstehenden Ansprüche, **dadurch gekennzeichnet, dass** die Einweg-Spritzeneinheit (34) weiter einen Schlauch (62) einschließt, der sich von dem Spritzenkörper (48) zu einem ersten Verbinder (64) erstreckt, und die endovaskuläre Vorrichtung einen zweiten Verbinder (20) an deren distalem Ende (16) einschließt, wobei der erste Verbinder (64) und der zweite Verbinder (20) verbunden werden können, um einen Fluidweg zwischen dem Spritzenkörper (48) und der endovaskulären Vorrichtung herzustellen.

6. Einrichtung (10) nach einem der vorstehenden Ansprüche, **dadurch gekennzeichnet, dass** das Gehäuse (32) mindestens ein Betätigungselement (68) für den Antriebsmotor (56) einschließt.

7. Einrichtung (10) nach einem der vorstehenden Ansprüche, **dadurch gekennzeichnet, dass** das Gehäuse (32) einen Drehzahlschalter (74) zum Einstellen einer Betriebsdrehzahl des Antriebsmotors (56) einschließt.

8. Einrichtung (10) nach einem der vorstehenden Ansprüche, **dadurch gekennzeichnet, dass** das Gehäuse (32) eine digitale Anzeige (78) zum Anzeigen von Betriebsinformationen bezüglich der Einrichtung (10) einschließt,
wahlweise, falls abhängig von Anspruch 2, **dadurch gekennzeichnet, dass** die Einrichtung (10) weiter eine Steuereinheit (82) einschließt, die dafür ausgebildet ist, den Betrieb des Antriebsmotors (56) und der digitalen Anzeige (78) zu steuern.

9. Einrichtung (10) nach Anspruch 8, **dadurch gekennzeichnet, dass** die digitale Anzeige (78) mindestens einen von einem Balloninflationsdruck und einem Volumen von aus dem Spritzenkörper (48) injizierter Flüssigkeit anzeigt.

10. Einrichtung (10) nach Anspruch 2, **dadurch gekennzeichnet, dass** das mindestens eine Betätigungselement (68) einen ersten Druckknopf (70) zum Zurückziehen des Kolbens (50) und einen zweiten Druckknopf (72) zum Herausziehen des Kolbens (50) einschließt.

11. Einrichtung (10) nach Anspruch 2, **dadurch gekennzeichnet, dass** der Antriebsmotor (56) ein Elektromotor ist.

12. Einrichtung (10) nach Anspruch 11, **dadurch gekennzeichnet, dass** die Einrichtung (10) weiter eine auf dem Gehäuse (32) getragene Batterie (80) einschließt, die zum Antreiben des Elektromotors angepasst ist.

13. Einrichtung (10) nach Anspruch 2, **dadurch gekennzeichnet, dass** der Antriebsmotor (56) ein Pneumatikmotor ist.

14. Einrichtung (10) nach Anspruch 2, **dadurch gekennzeichnet, dass** das Gehäuse (32) einen Sitz zum innerhalb des Gehäuses (32) in Position Halten des Spritzenkörpers (48) einschließt, während der Kolben (50) in dem Spritzenkörper (48) verlagert wird.

15. Einrichtung (10) nach Anspruch 2, **dadurch gekennzeichnet, dass** die Einrichtung (10) weiter eine Steuereinheit (82) einschließt, die dafür eingerichtet ist, den Betrieb des Antriebsmotors (56) und als Folge die Zurückziehung oder Herausziehung der Spritze in Bezug auf den Spritzenkörper (48) zu steuern.

## Revendications

1. Appareil (10), comprenant :
un dispositif endovasculaire ; et
un ensemble seringue (30) configuré pour être motorisé raccordé au dispositif endovasculaire
**caractérisé en ce que**
l'ensemble seringue (30) comprend
une unité (36) d'entraînement réutilisable incluant un moteur (56) d'entraînement et un boîtier (32), dans lequel le boîtier (32) est un boîtier tenu à la main qui est ergonomiquement adapté pour être tenu dans la main d'un médecin ; et
une unité (34) de seringue jetable, dans lequel le boîtier (32) inclut un couvercle (38) amovible et un compartiment (40) interne pour recevoir et maintenir l'unité (34) de seringue jetable et le moteur (56) d'entraînement.

2. Appareil (10) selon la revendication 1, **caractérisé en ce que** (a) l'unité (36) d'entraînement réutilisable inclut un moteur (56) d'entraînement présentant un pignon (58), dans lequel le moteur (56) d'entraînement et le pignon (58) sont maintenus dans le boîtier (32) et (b) l'unité (34) de seringue jetable inclut un corps (48) de seringue, un piston (50) reçu dans le corps (48) de seringue, un écrou (52) de tige porté sur le corps (48) de seringue et une tige (54) filetée portée sur le piston (50), dans lequel l'unité (34) de seringue jetable est maintenue dans le boîtier (32) et le pignon (58) s'engrène avec et entraîne l'écrou (52) de tige dans une première direction pour rétracter le piston (50) dans le corps (48) de seringue et dans une deuxième direction pour retirer le piston (50) du corps (48) de seringue.

3. Appareil (10) selon l'une quelconque des revendications précédentes **caractérisé en ce que** ledit dispositif endovasculaire est sélectionné dans un groupe de dispositifs consistant en un cathéter (12), un cathéter angiographique, un cathéter diagnostique, un cathéter de guidage, un microcathéter, un cathéter spécialisé, une gaine, un ballonnet d'angioplastie, un ballonnet à revêtement médicamenteux, un ballonnet spécialisé, un ballonnet d'embolectomie, un ballonnet de valvuloplastie, un stent expansible par ballonnet, un stent auto-expansible, un stent couvert expansible par ballonnet, un stent couvert auto-expansible, un dispositif d'administration de médicament, un dispositif d'embolisation, un dispositif d'athérectomie, un dispositif de franchissement de CTO, un dispositif de thrombectomie, un dispositif de gestion de produit de contraste, une ligne d'injection de produit de contraste, un dispositif d'aspiration de thrombus, un cathéter de dialyse, un cathéter à ligne centrale, un cathéter IV périphérique, un CCIP et un endoscope.

4. Appareil (10) selon la revendication 2, **caractérisé en ce que** l'écrou (52) de tige inclut une roue dentée intégrée qui s'engrène avec le pignon (58).

5. Appareil (10) selon l'une quelconque des revendications précédentes, **caractérisé en ce que** l'unité (34) de seringue jetable inclut en outre un tube (62) s'étendant du corps (48) de seringue jusqu'à un premier élément (64) de raccordement et le dispositif endovasculaire inclut un deuxième élément (20) de raccordement à l'extrémité (16) distale de celui-ci, grâce à quoi le premier élément (64) de raccordement et le deuxième élément (20) de raccordement peuvent être raccordés pour établir un trajet de fluide entre le corps (48) de seringue et le dispositif endovasculaire.

6. Appareil (10) selon l'une quelconque des revendications précédentes, **caractérisé en ce que** le boîtier (32) inclut au moins un actionneur (68) pour le moteur (56) d'entraînement.

7. Appareil (10) selon l'une quelconque des revendications précédentes, **caractérisé en ce que** le boîtier (32) inclut un commutateur (74) de vitesse pour ajuster une vitesse de fonctionnement du moteur (56) d'entraînement.

8. Appareil (10) selon l'une quelconque des revendications précédentes, **caractérisé en ce que** le boîtier (32) inclut un afficheur (78) numérique pour afficher des informations de fonctionnement concernant l'appareil (10), facultativement, s'il dépend de la revendication 2, **caractérisé en ce que** l'appareil (10) inclut en outre un dispositif (82) de commande configuré pour commander le fonctionnement du moteur (56) d'entraînement et de l'afficheur (78) numérique.

9. Appareil (10) selon la revendication 8, **caractérisé en ce que** l'afficheur (78) numérique affiche au moins l'une ou l'un d'une pression de gonflage de ballonnet et d'un volume de liquide injecté à partir du corps (48) de seringue.

10. Appareil (10) selon la revendication 2, **caractérisé en ce que** ledit au moins un actionneur (68) inclut un premier bouton-poussoir (70) pour rétracter le piston (50) et un deuxième bouton-poussoir (72) pour retirer le piston (50).

11. Appareil (10) selon la revendication 2, **caractérisé en ce que** le moteur (56) d'entraînement est un moteur électrique.

12. Appareil (10) selon la revendication 11, **caractérisé en ce que** l'appareil (10) inclut en outre une batterie (80) portée sur le boîtier (32) et adaptée pour alimenter le moteur électrique.

13. Appareil (10) selon la revendication 2, **caractérisé en ce que** le moteur (56) d'entraînement est un moteur pneumatique.

14. Appareil (10) selon la revendication 2, **caractérisé en ce que** le boîtier (32) inclut un siège pour maintenir le corps (48) de seringue en place à l'intérieur du boîtier (32) pendant que le piston (50) est déplacé dans le corps (48) de seringue.

15. Appareil (10) selon la revendication 2, **caractérisé en ce que** l'appareil (10) inclut en outre un dispositif (82) de commande configuré pour commander le fonctionnement du moteur (56) d'entraînement et, en conséquence, la rétraction ou le retrait de la seringue par rapport au corps (48) de seringue.
